# EUROPÄISCHE PATENTSCHRIFT

(11) **EP 1 599 727 B1**
(45) Veröffentlichungstag und Bekanntmachung des Hinweises auf die Patenterteilung: **14.05.2008**
(21) Anmeldenummer: 04716215.1
(22) Anmeldetag: 02.03.2004
(51) Int. Cl.: G01N 33/50, B01J 19/00

(54) **VERFAHREN ZUR HERSTELLUNG EINES TRÄGERS MIT IMMOBILISIERTEN VIREN UND VERWENDUNG EINES DERARTIGEN TRÄGERS**
METHOD FOR PRODUCING AN IMMOBILISED VIRUS CARRIER AND THE USE THEREOF
PROCEDE DE FABRICATION D'UN SUPPORT COMPORTANT DES VIRUS IMMOBILISES ET UTILISATION D'UN TEL SUPPORT

(30) Priorität: 04.03.2003 DE 10310252; 09.07.2003 DE 10332117
(43) Veröffentlichungstag der Anmeldung: 30.11.2005
(73) Patentinhaber: NMI NATURWISSENSCHAFTLICHES UND MEDIZINISCHES INSTITUT AN DER UNIVERSITÄT TÜBINGEN, 72770 Reutlingen (DE)
(72) Erfinder: VOLKMER, Hansjürgen, 72074 Tübingen (DE); WEISE, Frank, 72127 Kusterdingen (DE); JOOS, Thomas, 72074 Tübingen (DE)
(74) Vertreter: Otten, Hajo
(86) Internationale Anmeldenummer: PCT/EP2004/002054
(87) Internationale Veröffentlichungsnummer: WO 2004/078906

(56) Entgegenhaltungen:
- EP-A- 0 337 081
- WO-A-02/02226
- WO-A-89/01813
- WO-A-96/01900
- WO-A-97/45730
- WO-A-03/093462
- VOLKMER H ET AL: "Microarray-based assays as analytical tool for regenerative medicine." INTERNATIONAL JOURNAL OF ARTIFICIAL ORGANS, Bd. 26, Nr. 9, September 2003 (2003-09), Seite 850 XP009037232 & 1ST WORLD CONGRESS ON REGENERATIVE MEDICINE; LEIPZIG, GERMANY; OCTOBER 22-24, 2003 ISSN: 0391-3988

## Beschreibung

Die Erfindung betrifft ein Verfahren zur Herstellung eines Trägers mit einer funktionalisierten Oberfläche, ferner Träger, die durch dieses Verfahren hergestellt werden, sowie Verwendungen der durch dieses Verfahren hergestellten Träger.

Träger mit einer funktionalisierten Oberfläche finden insbesondere im Gebiet der Medizintechnik und der Bioanalytik ihre Anwendung, sowie insbesondere dort, wo der Bedarf an schnellen, reproduzierbaren und empfindlichen Analyseverfahren besteht. Verfahren zur Herstellung von Trägern mit funktionalisierten Oberflächen sind im Stand der Technik hinreichend bekannt.

Insbesondere für analytische Methoden in der Biologie und für die medizinische Diagnostik spielen heutzutage Träger mit funktionalisierten Oberflächen eine herausragende Rolle. Bei den Trägern handelt es sich um miniaturisierte, mikrostrukturierte Funktionselemente mit einer Vielzahl von biologischen und/oder technischen Komponenten, z.B. mit auf einer Oberfläche immobilisierten Biomolekülen, die als spezifische Interaktionspartner dienen können, sowie mit einer Matrix bzw. einem Trägermaterial. Die "funktionalisierte" Oberfläche des Trägers weist dabei in der Regel Moleküle mit funktionellen Gruppen auf, welche an "Fängermoleküle" binden, die mit anderen (Bio-)Molekülen, den "Liganden", in Wechselwirkung treten.

Eine Vielzahl von Biomoleküle ist dabei auf den Trägeroberflächen angeordnet, so dass eine Vielzahl verschiedener (bspw. genetischer) Informationen parallel und aus einer Probe untersucht werden kann.

Biomoleküle, die als solche bindenden Agenzien auf den Trägern angeordnet sind, sind in der Biotechnologie zu einem wichtigen Werkzeug geworden. Beispiele für Fängermoleküle, die auf Trägern als "Spots" angeordnet werden, sind bspw. Nucleinsäuren, wie DNA oder RNA, ferner Proteine oder Peptide, wie bspw. Antigene, Antikörper und Rezeptoren, Polysacharide, Lectine oder sogar ganze Zellen, wobei diese Substanzen in einer bestimmten Anordnung ("Arrays") auf einer festen Trägeroberfläche oder in einem virtuellen Array auf adressierbaren beads angeordnet sind. Derartige Arrays können bspw. bei der Entdeckung von Genen, der Genom-Forschung, in der Diagnostik, Bioanalytik und bei dem Screenen nach neuen bioaktiven Verbindungen eingesetzt werden. Eine wichtige Verwendung der Arrays ist die Analyse der differentiellen Genexpression, bei welcher die Expression von Genen in unterschiedlichen Zellen, typischerweise die zu untersuchende Zelle im Vergleich zu einer Kontrolle, verglichen wird. Dabei werden die Unterschiede in der Expression verschiedener Gene identifiziert. Weiterhin ist die Messung vieler verschiedener Liganden aus geringen Probenmengen wichtig.

Die genannten Arrays, bzw. deren Verwendung sind demnach moderne Verfahren, mit denen eine weitgehend automatisierte und miniaturisierte Parallelanalyse von Einzelprozessen möglich ist.

Ein anderes Anwendungsbeispiel für die oben erwähnten Träger mit immobilisierten Biomolekülen ist bspw. die Verwendung solcher Träger zum Nachweis, zur Isolierung oder Charakterisierung von Zellen in einer Lösung. So kann bspw. untersucht werden, welche Zellen in einer Probe an bestimmte immobilisierte Biomoleküle binden und welche nicht, wobei als Biomoleküle z.B. Nucleinsäuren oder Proteine eingesetzt werden können.

Eine weitere Möglichkeit, Träger mit immobilisierten Biomolekülen in der Biotechnologie einzusetzen, ist bspw. das Verfahren, wie es in der US-Patentanmeldung US 2002/0006664 von Sabatini beschrieben ist. Darin wird ein Verfahren und ein Träger offenbart, bei welchem zunächst DNA-Bruchstücke sowie in Plasmidvektoren klonierte DNA auf einer Trägerobefläche als Spots angeordnet und immobilisiert werden. In einem nächsten Schritt werden Zellen auf diese Trägeroberfläche ausgesät und die auf der Trägeroberfläche immobilisierte DNA wird anschließend in die Zellen eingeschleust, ein Vorgang der von den Erfindern dieses Verfahrens als "reverses Transfektions-Verfahren" bezeichnet wird.

Ein großer Nachteil dieses bekannten Verfahrens zur reversen Transfektion ist jedoch, dass die Zellen nicht nur an die immobilisierten Spots binden, sondern die Trägeroberfläche auch außerhalb der Spots besiedeln. Demnach müssen im Stand der Technik in einem weiteren Schritt stets die Zellen identifiziert werden, welche mit dem genetischen Material in Kontakt gekommen sind.

Ein weiterer Nachteil ist darin zu sehen, dass nur solche Zellen verwendet werden können, die mit Plasmiden leicht transfizierbar sind.

Ferner besteht allgemein hier die große Gefahr, dass bestimmte Zellen aus einem Zellgemisch nicht selektiv isoliert werden können, da verschiedene Zellen zwischen den Spots binden können.

Vor diesem Hintergrund ist es Aufgabe der vorliegenden Erfindung, einen Träger mit neuen Biomolekülen sowie ein Verfahren zu seiner Herstellung bereitzustellen, mit welchem die o.g. Nachteile überwunden werden können.

Diese Aufgabe wird erfindungsgemäß durch ein Verfahren zur Herstellung eines Trägers mit einer funktionalisierten Oberfläche und mit auf der funktionalisierten Oberfläche immobilisierten, vorzugsweise infektiösen Viren gelöst, welches die folgenden Schritt aufweist:
(a) Bereitstellen eines Trägermaterials;
(b) Beschichten von zumindest einer Oberfläche des Trägermaterials zur Erzeugung einer für die Immobilisierung von Viren geeigneten funktionalisierten Oberfläche;
(c) Aufbringen einer Lösung, welche vorzugsweise infektiöse Viren enthält, auf die funktionalisierte Oberfläche;
(d) Anhaftenlassen der Viren an die funktionalisierte Oberfläche für einen Zeitraum von vorzugsweise 10 Minuten bis 36 Stunden;
(e) ggf. Bedecken der Oberfläche mit einem Fluid.

Ferner wird die Aufgabe gelöst durch einen Träger mit einer für die Immobilisierung von Viren geeigneten funktionalisierten Oberfläche und mit auf der funktionalisierten Oberfläche immobilisierten, vorzugsweise infektiösen Viren, sowie durch Verwendungen eines Trägers mit immobilisierten Viren, die infektiös oder nicht-infektiös sein können.

Die der Erfindung zugrundeliegende Aufgabe wird dadurch vollkommen gelöst.

Die Erfinder konnten in eigenen Versuchen einen Träger mit einer für die Immobilisierung von Viren geeigneten funktionalisierten Oberfläche und mit an der funktionalisierten Oberfläche immobilisierten Viren herstellen, mit welchem Zellen gezielt an Spots auf dem Trägermaterial immobilisiert werden konnten. Die Erfinder konnten dabei zeigen, dass sich der Träger, bzw. die Immobilisierung der Viren, als stabil erwies, und dass die Viren noch über einen längeren Zeitraum infektiös waren und Zellen binden konnten.

Im Stand der Technik war es bisher nicht möglich, infektiöse Viren an Oberflächen eines Trägers zu immobilisieren. Vielmehr galt allgemein eine über viele Tage andauernde Lagerung bzw. Immobilisierung von Viren anders als in infizierten Zellen als selbst bei tiefen Temperaturen instabil und somit als unmöglich (siehe bspw. Davis et al., "Microbiology", 3. Auflage, 1985, S. 1094). Mit dem erfindungsgemäßen Verfahren können nun Träger hergestellt werden, auf welchen infektiöse Viren immobilisiert vorliegen. Dabei konnten die Erfinder ferner zeigen, dass die erfindungsgemäß hergestellten Träger über längere Zeit bei Raumtemperaturen gelagert werden können und funktionsfähig bleiben, die Viren also ihre Bindungsfähigkeit und Infektiösität behalten.

Im Stand der Technik waren bisher keine Träger mit derart immobilisierten Viren bekannt, die nach der Immobilisierung noch in der Lage sind, an Zellen zu binden. Schon Arrays von nichtinfektiösen Viren bieten viele neue Anwendungsmöglichkeiten, bei denen die Bindung zwischen Viren und Zellen untersucht wird.

Bei den neuen Trägern ist weiter von Vorteil, dass die Viren den Gentransfer in bzw. die Transduktion von Zellen ermöglichen, die schwer oder gar nicht transfizierbar sind. Dies sind z.B. Zellen, die von hoher Relevanz sind wie bspw. Primäre Zellen. Mit den erfindungsgemäß hergestellten Trägern ist es somit möglich, im Vergleich zur Plasmidtransfektion nach Sabatini das Spektrum an Zellen deutlich zu erhöhen, in die Gene transferiert werden können.

Die neuen Träger ermöglichen es, dass Zellen über Oberflächenrezeptoren an immobilisierte Viren binden können, die wiederum das Vermögen haben, diese Zellen zu infizieren. Wenn die Virussuspension in Reihen und Spalten in Arrayform aufgetragen wird, können in miniaturisierten und parallelisierten Formaten Zellen infiziert werden. Rekombinante immobilisierte Viren erlauben entsprechend einen miniaturisierten und parallelisierten Gentransfer sowie Genexpression in durch andere gentechnische Methoden nur schwer manipulierbaren Zellen.

Die Viren sind dabei in einzelnen Spots angeordnet, wobei verschiedene Spots verschiedene Virustypen oder -arten enthalten können. In einem Spot ist für die Transduktion von Zellen dabei die PFU, also die plaque forming units, die angibt, wie viele infektiöse Viruspartikel vorhanden sind, zu beachten.

Es ist mit den neuen Trägern erstmals auch möglich, Arrays von infektiösen aber auch nicht-infektiöse Viren bereitzustellen, die verschiedene Zellen spezifisch binden können. Dabei können auch verschiedene Virustypen und -arten immobilisiert werden, deren Bindefähigkeit an Zellen aus einer Probe untersucht wird, wobei wegen der Miniaturisierung und Parallelisierung mit geringen Probenmengen gearbeitet werden kann. "Träger" bedeutet im Rahmen der vorliegenden Anmeldung sowohl einen planaren Träger mit physikalisch zusammenhängender Oberfläche als auch ein virtuelles Array aus sog. Beads oder Mikrobeads, die zudem markiert und somit adressierbar sein können.

"Trägermaterial" bedeutet vorliegend einen Träger mit zumindest einer Oberfläche, auf welcher allgemein Biomoleküle wie Nukleinsäuren, Proteine, Zellen oder Viren immobilisiert werden können. Der Fachmann wird erkennen, dass hierbei eine Vielzahl an Trägermaterialien, die im Stand der Technik - bspw. im Gebiet der Bioanalytik - eingesetzt werden, für das erfindungsgemäße Verfahren geeignet sind.

"Funktionalisierte Oberfläche" bedeutet vorliegen jede Oberfläche, welche Moleküle mit funktionellen Gruppen aufweist, über welche die Bindung von Biomolekülen - bspw. Nukleinsäuren, Proteine, ganze Zellen usw. - vermittelt wird.

"Eine für die Immobilisierung von Viren geeignete funktionalisierte Oberfläche" bedeutet vorliegend eine Oberfläche mit einer Beschichtung, die einerseits die Viren bindet, also immobilisiert, es andererseits aber ermöglicht, dass die Viruspartikel in oder an der Oberfläche noch leicht für Zellen zugänglich sind. Mit anderen Worten, die Viren sind noch so beweglich, dass sie sterisch an die Zelloberflächenrezeptoren gelangen können und sich gegenseitig dabei nicht behindern. Die funktionalisierte Oberfläche ermöglicht dies nach Erkenntnis der Erfinder insbesondere dann, wenn sie eine Art Maschenwerk bildet, in dem die Viruspartikel fest aber beweglich gebunden werden. Dies ist nach der überraschenden Erkenntnis der Erfinder beispielsweise mit einer Beschichtung aus Nitrocellulose oder Materialien mit ähnlichen Eigenschaften zu erreichen, wo die Viruspartikel bspw. über eine elektrostatische Wechselwirkung an einzelne Fasern gebunden werden, die eine gewisse Beweglichkeit in der Beschichtung aufweisen. Es sind aber auch andere Mechanismen einsetzbar, die es den immoblisierten Viren, die als Aggregate vorliegen können, erlauben, an die Zellen zu binden. Diese Bindung muss es im Falle eines gewünschten Gentransfers u.U. erlauben, dass die Viren internalisiert werden.

Die Viruspartikel sollten dazu nicht-kovalent gebunden sein, wie es bspw. mit einer Beschichtung aus Nitrocellulose, die ggf. zusätzlich mit Collagen beschichtet ist, oder mit Beschichtungen erreicht wird, die eine dauerhafte positive Ladung auf der Oberfläche bewirken, wie dies bspw. bei den kommerziell erhältlichen "Superfrost (TM) Plus" Trägern der Fall ist, die von EMS, Electron Microscopy Sciences in ... vertrieben werden. Ferner sind mit Aminosilan beschichtete Oberflächen verwendbar, wie bspw. bei den kommerziell erhältlichen "GAPS II" Coated Slides", die von Corning Inc, Acton, Massachusetts, USA, vertrieben werden.

"Infektiös" bedeutet vorliegend, dass die Viren vor und nach der Immobilisierung in der Lage sind, Zellen zu infizieren, also das in den Viren enthaltene genetische Material, DNA oder RNA, bei Kontakt mit Zellen in diese einzuschleusen, sollte dies das Ziel einer Verwendung eines durch das erfindungsgemäße Verfahren hergestellten Trägers sein. Dabei ist in einem Spot auch darauf zu achten, dass die PFU hinreichend hoch ist, so dass genügend Bindungspunkte für die Adhäsion der an den immobilisierten Viren zu immobilisierenden Zellen vorhanden sind. "Nicht-infektiös" bedeutet dagegen, dass die Viren in der Lage sind, Zellen zu binden, ohne jedoch ihr genetisches Material in die gebundenen Zellen einzuschleusen.

Der erfindungsgemäße Träger kann zu verschiedenen Zwecken eingesetzt werden, insbesondere zur Untersuchung der Wechselwirkung von in einer Lösung befindlichen Zellen mit den immobilisierten Viren. Mit einem nach dem erfindungsgemäßen Verfahren hergestellten Träger kann bspw. erreicht werden, dass - nach Inkubation einer Zellen-enthaltenden Lösung - diese Zellen auf dem Träger auf Spots fokussiert werden, also nur an Stellen binden, an denen Viren immobilisiert vorliegen.

Die Erfinder konnten ferner in eigenen Versuchen zeigen, dass über die stabil immobilisierten Viren fremdes genetisches Material in Zellen eingeschleust werden konnte, welche an die Viren binden. Dies zeigte, dass die Viren durch das erfindungsgemäße Verfahren auch im immobilisierten Zustand infektiös bleiben.

Nach Immobilisierung der Viren auf der Trägeroberfläche kann der Träger zur Verhinderung der Austrocknung mit einem geeigneten Fluid bedeckt werden, bspw. mit Phosphat-gepufferter Salzlösung (PBS), Polyethylenglykol (PEG), Protein- oder Polysacharidlösungen.

In einer Ausführungsform ist bevorzugt, wenn das Trägermaterial ein Material aufweist, das als Oberfläche für Zellkulturen geeignet ist und vorzugsweise aus der Gruppe umfassend Zellkulturkunststoffe, Glas, Silicon, Polystyrol, ausgewählt ist.

Derartige Trägermaterialien haben sich im Stand der Technik als bewährt erwiesen und werden im Fachgebiet vielfältig angewandt. "Trägermaterial" im Sinne der vorliegenden Erfindung kann somit jedes Material sein, welches sich zur Durchführung dieses Verfahrens eignet, und welche im Stand der Technik für die Herstellung von ähnlichen Trägern verwendet werden, bspw. auch markierte Mikrobeads oder planare Zellkulturträger.

Insbesondere ist bevorzugt, wenn bei dem Verfahren das Trägermaterial mit einem Polykation, bspw. mit poly-L-Lysin oder Aminosilan vorbeschichtet ist, wobei Aminosilan auch selbst bereits eine für die Immobilisierung von Viren geeignete Oberfläche ist.

Derart beschichtete Oberflächen sind im Stand der Technik hinreichend bekannt und werden vielfach auch in medizinischen Fachgebieten verwendet. So kann bei dem erfindungsgemäßen Verfahren als Trägermaterial bspw. ein mit poly-L-Lysin vorbeschichteter gläserner Objektträger eingesetzt werden.

Gläserne Objektträger werden bspw. von der Firma Sigma, Taufkirchen, Deutschland, unter der Bezeichnung PolyPrep vertrieben.

Ferner ist bevorzugt, wenn die Lösung, welche die Viren enthält, Glycerol aufweist.

Die Verwendung von Glycerol hat den Vorteil, dass die Viren im feuchten Zustand aktiv gehalten werden. Neben Glycerol sind dabei auch glycerolähnliche Verbindungen zum Einsatz in dem Verfahren denkbar, wie bspw. Trehalose, Sacharose sowie allgemein Stoffe, die Proteinlösungen stabilisieren können.

Ferner ist bevorzugt, wenn die in der Lösung vorliegenden Viren Adenoviren oder Adeno-assoziierte Viren (AAV) sind. Hierbei sind insbesondere Adenoviren vom Serotyp 5 bevorzugt.

Die Erfinder haben erkannt, dass es bspw. durch die Verwendung von Adenoviren vom Serotyp 5 möglich ist, Zellen an die auf dem Trägermaterial immobilisierten Viren zu fokussieren. Im Stand der Technik galt bisher die Lagerung oder Immobilisierung von infektiösen Adenoviren außerhalb von Zellen oder lebenden Wirten als nicht möglich.

In einer Weiterbildung des erfindungsgemäßen Verfahrens ist bevorzugt, wenn die Viren gentechnisch verändert sind.

Dies bedeutet, dass in die genetische Information der Viren Viren-fremde Sequenzen eingebaut sein können, die in den immobilisierten Zellen exprimiert werden. Die gentechnische Veränderung des Viren-Genoms kann dabei mit im Stand der Technik gebräuchlichen Verfahren durchgeführt werden. Die Viren sind dann sog. Vektoren, wobei in diesem Zusammenhang die Viren ausgewählt sind aus der Gruppe: Adenoviren und AAV.

Insbesondere ist bevorzugt, wenn die Viren eine Sequenz aufweisen, die für das Green-fluorescent-Protein codiert. Dieses Protein wurde von Prasher et al., "Primary structure of the Aequorea victoria green-fluorescent protein", Gene 111:229-293 (1992), zum ersten Mal kloniert und hat sich in den verschiedensten Gebieten der Biologie und Medizintechnik als herausragendes Reportergen bewährt, das anzeigt, ob eine Übertragung von genetischem Material aus den Viren in die Zellen erfolgt ist.

Als Reportergene geeignet sind ferner bspw. β-Galaktosidase, Luciferase, alkalische Phosphatase, Peroxidase.

Ferner ist bevorzugt, wenn als Virus-enthaltende Lösung ein Überstand von Virus-infizierten Zellen eingesetzt wird, und insbesondere ein Überstand, der vor dem Aufbringen auf die funktionalisierte Oberfläche gereinigt und/oder aufkonzentriert wird.

Die Erfinder konnten in eigenen Versuchen zeigen, dass die Immobilisierung sich als besonders stabil erwies, wenn die Viren-Lösung, die aus dem Überstand von infizierten Zellen gewonnen wurde, vor der Aufbringung gereinigt und/oder aufkonzentriert wurde. Eine Aufreinigung des Viren-Materials, bzw. des Zellkulturüberstandes kann bspw. durch eine Dichtegradientenzentrifugation, insbesondere eine Cäsiumchlorid (CsCl)-Dichtegradientenzentrifugation, erreicht werden. Auch die gereinigten und anschließend immobilisierten Viren waren trotz der oben beschriebenen kruden Behandlung auch über einen längeren Zeitraum hinweg noch infektiös. Dies war nach dem bisherigen Stand der Technik nicht zu erwarten.

In einer Ausführungsform des erfindungsgemäßen Verfahrens ist bevorzugt, wenn vor Schritt (c) Rinderserumalbumin auf die funktionalisierte Oberfläche aufgebracht wird.

Die Erfinder haben erkannt, dass durch diese Maßnahme die Stabilität und Infektiosität der Viren weiter vorteilhaft verlängert werden konnte.

In einer Weiterbildung des erfindungsgemäßen Verfahrens ist bevorzugt, wenn der Zeitraum, in dem die Viren auf der funktionalisierten Oberfläche anhaften, 15 bis 20 Stunden beträgt.

Dieser von den Erfindern in einigen Versuchen gewählte Zeitraum erwies sich für eine ausreichende Anhaftung der Viren als geeignet, dennoch sind auch kürzere Zeiträume für eine Beschleunigung des Verfahrens möglich.

Ferner ist bei dem erfindungsgemäßen Verfahren bevorzugt, wenn die Temperatur, bei welcher die Viren an die funktionalisierte Oberfläche anhaften, von -20°C bis +37°C, und insbesondere von 0°C bis +10°C beträgt.

In eigenen Versuchen der Erfinder wurde dabei ein niedriger Temperaturbereich ausgewählt, um eine Stabilisierung der Viren und somit deren Infektiosität zu gewährleisten. Die Temperatur liegt dabei vorzugsweise oberhalb des Gefrierpunktes der Suspension.

Die Erfindung betrifft ferner Träger mit einer für die Immobilisierung von Viren geeigneten funktionalisierten Oberfläche und mit auf der funktionalisierten Oberfläche immobilisierten, vorzugsweise infektiösen Viren, und insbesondere derartige Träger, die nach dem erfindungsgemäßen Verfahren hergestellt sind. Die Erfindung betrifft ferner die Verwendung eines Trägers mit einer für die Immobilisierung von Viren geeigneten funktionalisierten Oberfläche und mit auf der funktionalisierten Oberfläche immobilisierten Viren zur Untersuchung von medizinischen Proben.

Bei dieser erfindungsgemäßen Verwendung wird eine medizinische Probe mit einem Träger in Kontakt gebracht, auf dessen Oberfläche sich immobilisierte Viren befinden und anschließend die Reaktion zwischen Substanzen, die sich in der medizinischen Probe befinden, mit den auf der Trägeroberfläche immobilisierten Viren untersucht.

Dieses Verwendung bietet den Vorteil, dass medizinische Proben bspw. mit virusspezifischen Nachweismolekülen - in der Regel Antikörper - versetzt werden können. Diese Reaktion kann anschließend im Rahmen eines Hochdurchsatzverfahrens auf die Bindung an die immobilisierten Viren untersucht werden.

Unter "medizinischer Probe" wird in diesem Zusammenhang eine Probe verstanden, die für diagnostische oder therapeutische Zwecke relevante Zellen, Antikörper oder allgemein Proteine enthalten, deren Bindungsfähigkeit an die immoblisisierten Viren getestet werden soll.

Ein Einsatzgebiet der neuen Träger ist dabei das Durchmustern von Bibliotheken von gentechnisch veränderten Viren, insbesondere Adenoviren, auf ihre Zelladhäsionsfähigkeit. Diese Viren können dabei auf einen veränderten Tropismus gescreent werden, wenn also die Hüllproteine genetisch verändert werden, um die Viren für ein neues Zielgewebe spezifisch zu machen. Diese Viren werden insbesondere in der Gentherapie eingesetzt, wenn in bestimmte Zielgewebe genetisches Material eingeschleust werden soll, wobei das Nachbargewebe für die Transduktion nicht zugänglich sein soll.

Wenn die Viren Phagen sind, können die neuen Träger auch für Phagendisplay eingesetzt werden. Auf der Hülle von Bakteriophagen werden dabei z.B. unterschiedliche Antikörperfragmente exprimiert, deren Bindungsfähigkeit an Zellen oder an Proteine getestet wird.

Ferner betrifft die Erfindung die Verwendung eines Trägers mit einer für die Immobilisierung von Viren geeigneten funktionalisierten Oberfläche und mit auf der funktionalisierten Oberfläche immobilisierten Viren zur Charakterisierung von Zellen und/oder zur Untersuchung von Zelladhäsion.

Auch bei dieser Verwendung wird eine Zellsuspension mit einem Träger in Kontakt gebracht, auf dessen Oberfläche Viren immobilisiert sind, und anschließend die Reaktion der in der Suspension vorliegenden Zellen mit den Viren analysiert.

Hier wird z.B. die Zelladhäsion getestet, entweder für bestimmte Zellen gegen viele verschiedene Viren oder für bestimmte Viren gegen viele verschieden Zellen.

Die Charakterisierung von Zellen ist insbesondere für analytische Fragestellungen bedeutsam. Vorteilhaft bei der erfindungsgemäßen Verwendung ist hierbei die Spezifität der Wechselwirkung der viralen Liganden mit den zellulären Rezeptoren. So können ggf. aus einem Zellgemisch die zu untersuchenden Zellen, bzw. diejenigen Zellen, die an die immobilisierten Viren binden, ohne Schwierigkeiten auch aus kleinen Ausgangsmengen angereichert werden.

In dieser Verwendung nachgeschalteten Verfahren können die angereicherten Zellen für eine Vielzahl weiterführender Untersuchungen eingesetzt werden, wie bspw. zur Untersuchung des Stoffwechsels oder der Reaktion auf äußere Reize, Umwelteinflüsse, etc.

Die Erfindung betrifft ferner die Verwendung eines Trägers mit einer für die Immobilisierung von Viren geeigneten funktionalisierten Oberfläche und mit auf der funktionalisierten Oberfläche immobilisierten, infektiösen Viren zum Transfer genetischen Materials in Zellen.

Hier wird zusätzlich zu der oben bereits angesprochenen Zelladhäsion auch noch der Gentransfer getestet bzw. ausgenutzt.

Insbesondere ist diese Verwendung bevorzugt, wenn folgende Schritte durchgeführt werden:
(a) Bereitstellen eines Trägers mit einer für die Immobilisierung von Viren geeigneten funktionalisierten Oberfläche und mit auf der funktionalisierten Oberfläche immobilisierten infektiösen Viren, und
(b) In-Kontakt-Bringen des Trägers mit einer Suspension, welche Zellen enthält.

Diese Verwendung bietet den Vorteil, dass ein automatisierter, miniaturisierter, paralleler Gentransfer ermöglicht wird. Durch die Immobilisierung von Viren können auch primäre Zellen mit genetischem Material transfiziert werden. Von Interesse sind hierbei bspw. auch Gene, die nicht aus dem Genrepertoire der Viren stammen: So kann bspw. in die Viren Genmaterial eingefügt werden, die Viren anschließend an die Trägeroberfläche immobilisiert werden, und anschließend die zu transfizierenden Zellen auf den Träger gegeben werden.

Mit der erfindungsgemäßen Verwendung besteht die Möglichkeit, jegliche genetische Information in die Zielzellen einzubringen, denn nach Anheftung der Zellen an die immobilisierten Viren schleusen diese ihr Genmaterial in die anhaftenden Zellen ein. Vorteilhafterweise werden dadurch nur diejenigen Zellen transfiziert, die auch an immobilisierte Viren anhaften. Ein derartiges Verfahren ist insbesondere für Zelltypen von Bedeutung, die dem klassischen, physikalischen Gentransfer nicht zugänglich sind.

Der Nachweis des Gentransfers kann bspw. durch die Reaktion der Zelle auf den Gentransfer mittels verschiedener Tests untersucht werden, wie bspw. visuell über Veränderungen der Morphologie, enzymatisch über Veränderungen des Stoffwechsels, immunologisch über Veränderung der den Zellstatus kennzeichnenden Zellprodukte, über die Expression von Reportergenen usw.

Werden anstelle der natürlicherweise vorkommenden Viren solche verwendet, die gentechnisch verändert worden sind, besteht die Möglichkeit, jedwede genetische Information in die Zelle einzubringen.

Die Transfektion von Zellen mit DNA ist zwar bereits von Sabatini et al. (siehe US-Patentanmeldung US 2002/0006664 A1) beschrieben worden. Hier jedoch erfolgt die Transfektion der Zellen durch Verwendung von DNA, welche in einem Expressionsvektor vorliegt, wobei eine Mischung mit einem Trägerprotein vorzugsweise Gelatine) verwendet wird, welche auf dem Trägermaterial immobilisiert wird. Eine Immobilisierung von Viren, welche zu transduzierende DNA aufweisen, ist nicht beschrieben oder vorgeschlagen.

Bei Ausführungsformen der erfindungsgemäßen Verwendungen ist bevorzugt, wenn die Zellen eukaryontische Zellen sind, und zwar insbesondere primäre Zellen oder immortalisierte Zellen.

Die erfindungsgemäße Verwendung zum Gentransfer ist vor allem bei primären Zellen eine herausragende Methode, um diese Zellen zu transduzieren, da der Gentransfer in diese Zellen mit herkömmlichen Verfahren bekanntermaßen schwierig ist.

Andererseits können auch Bakteriophagen immobilisiert und bspw. Bakterien transduziert werden. Als Anwendung ist hier die Expressionskontrolle beim Screenen auf erfolgten Gentransfer zu nennen. Es können aber auch Bibliotheken von Phagen auf ihre Fähigkeit untersucht werden, an Zelloberflächenrezeptoren zu binden, wie es bspw. für Phagendisplay eingesetzt werden kann.

Allgemein ist bevorzugt, wenn für die erfindungsgemäßen Verwendungen ein Träger eingesetzt wird, der nach dem oben erwähnten erfindungsgemäßen Verfahren hergestellt ist.

Die Erfindung betrifft ferner allgemein ein Verfahren zur Einschleusung von DNA oder RNA in eine pro- bzw. eukaryontische Zelle, mit den Schritten:
(a) Aufbringen einer Virus-enthaltenden Lösung auf einen Träger mit einer für die Immobilisierung von Viren geeigneten funktionalisierten Oberfläche,
(b) Anhaftenlassen der Lösung auf der Oberfläche, und
(c) Aufbringen von einer Suspension, welche Zellen enthält, auf die Oberfläche, um die Zellen mit der in den Viren enthaltenden DNA oder RNA zu transduzieren.

Das erfindungsgemäße Verfahren bietet die herausragende Möglichkeit, genetisches Material schnell und effizient in Zellen einzuschleusen. Dieses Verfahren ist im Vergleich zu dem bekannten Verfahren, bei welchem die zu transfizierende DNA direkt auf dem Träger immobilisiert vorliegt, günstiger, da vorliegend die Zellen spezifisch auf die Spots fokussiert werden, d.h. nur an Stellen auf dem Träger binden, an welchen die Viren immobilisiert sind.

Hierbei ist insbesondere bevorzugt, wenn ein Träger verwendet wird, der nach dem erfindungsgemäßen Verfahren hergestellt ist.

Die Erfinder konnten durch eigene Versuche zeigen, dass durch erfindungsgemäß hergestellte Träger mit immobilisierten Viren genetisches Material in Zellen eingeschleust werden kann, welche bspw. in einer Suspension auf den Träger gegeben werden. Ein derartiges Verfahren und ein dazu einzusetzender Träger waren im Stand der Technik bisher nicht bekannt, da die Immobilisierung von Viren und eine stabile Lagerung bisher nicht möglich war.

Die Erfindung betrifft ferner ein Verfahren zum Lagern von vorzugsweise infektiösen Viren auf einem Träger mit einer für die Immobilisierung von Viren geeigneten funktionalisierten Oberfläche und insbesondere auf einem Träger, der nach dem erfindungsgemäßen Verfahren hergestellt ist.

Die Erfinder konnten in eigenen Versuchen zeigen, dass es möglich ist, Viren für einen längeren Zeitraum auf einem Träger stabil zu immobilisieren, ohne dass sie ihre Fähigkeit, an Zellen zu binden, oder ihre Infektiosität verlieren. Eine Lagerung von infektiösen Viren außerhalb von Zellen oder entsprechenden lebenden Wirten, war - wie oben erwähnt - bisher im Stand der Technik nicht bekannt.

Es versteht sich, dass die vorstehend genannten und die nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

Ausführungsbeispiele der Erfindung werden den nachfolgenden Beispielen und den beigefügten Figuren dargestellt und erläutert. Es zeigen:
- Fig. 1a: eine schematische Darstellung eines erfindungsgemäßen Trägers in einer perspektivischen Ansicht;

- Fig. 1b: eine schematische Darstellung des erfindungsgemäßen Trägers aus Fig. 1a in Draufsicht;
- Fig. 2a: fluoreszenzmikroskopische Aufnahmen von infizierten bzw. nicht infizierten HEK293-Zellen nach 7 Tagen;
- Fig. 2b: fluoreszenzmikroskopische Aufnahmen infizierter bzw. nicht infizierter HEK293-Zellen nach 14 Tagen;
- Fig. 3: eine Kollage verschiedener Ausschnitte der Fluoreszenz eines Spots einer Kammer;
- Fig. 4: fluoreszenzmikroskopische Aufnahmen infizierter primärer Zellen; und
- Fig. 5-9: Aufnahmen wie in Fig. 2a und 2b, jedoch für weitere Oberflächen.

### Beispiel 1

### Funktionalisierung der Oberfläche des Trägermaterials

Kommerziell erhältliche, mit poly-L-Lysin vorbeschichtete gläserne Objektträger (PolyPrep, Sigma, Taufkirchen, Deutschland) wurden mit Nitrocellulose beschichtet. Dazu wurden die Objektträger für 2 Minuten in ein mit in Methanol gelöster Nitrocellulose (2,5 mg/ml) gefülltes Tauchbad gegeben. Anschließend wurden die Träger unter sterilen Bedingungen getrocknet. Auf diese Weise wurde die funktionalisierte Oberfläche für die nachfolgende Immobilisierung der Viren hergestellt.

### immobilisierung der Viren

### a) Beschreibung des verwendeten Virenmodells

Als Modellsystem wurden kommerziell erhältliche, durch gentechnische Veränderung in ihrer Vermehrung auf spezielle Hilfszellen (HEK293) angewiesene Adenoviren des Serotyps 5 (AdEasy-1, Stratagene, La Jolla, USA) verwendet. Diese Viren weisen die Eigenschaft auf, ein breites Spektrum von Säugerzellen infizieren zu können, sie vermögen sich jedoch nicht in diesen Zellen zu vermehren. In diese Viren wurde über eine weitere gentechnische Veränderung ein fremdes Gen eingebracht, welches ein durch Fluoreszenz nachweisbares Produkt liefert, das Green Fluorescent Protein (im Folgenden GFP). Sowohl das Virus als auch das GFP finden in molekularbiologischen Laboratorien standardmäßig Verwendung. Das verwendete Virus wird im Folgenden AdEasy-CMV-GFP bezeichnet.

### b) Präparation der Viren

Folgende Viruspräparationen wurden von den Erfindern zum Einsatz gebracht: Zum einen wurde virushaltiger Überstand infizierter HEK293-Zellen verwendet, zum anderen Viren, die aus diesem Zellüberstand über eine Dichtegradientenzentrifugation (Cäsiumchlorid-Gradient) aufgereinigt wurden. HEK293 (human embryonic kidney) -Zellen sind bspw. bei der Deutschen Sammlung für Mikroorganismen und Zellkulturen (DSMZ) unter der DSMZ-Nummer ACC305 erhältlich. Die beiden Viruspräparationen wurden jeweils 1:1,3 mit Auftragspuffer verdünnt. Bei dem Auftragspuffer handelt es sich um eine 80-%ige Lösung von Glycerol in phosphatgepufferter Salzlösung (PBS).

### c) Immobilisierung der Viren in Array-Anordnung

Im Modellversuch wurden auf den Objektträgern (siehe oben) 8 beschickbare Kammern eingerichtet, indem desinfizierte Kunststoffgitter (FlexiPERM, Vivascience, Göttingen) sanft auf die Oberfläche gedrückt wurden. Zur Verhinderung von Kontakten zwischen den Kammern wurde die Auflagefläche der Gitter zuvor mit Petrolatum (Vaseline, Lever Fabergé, Hamburg) imprägniert.

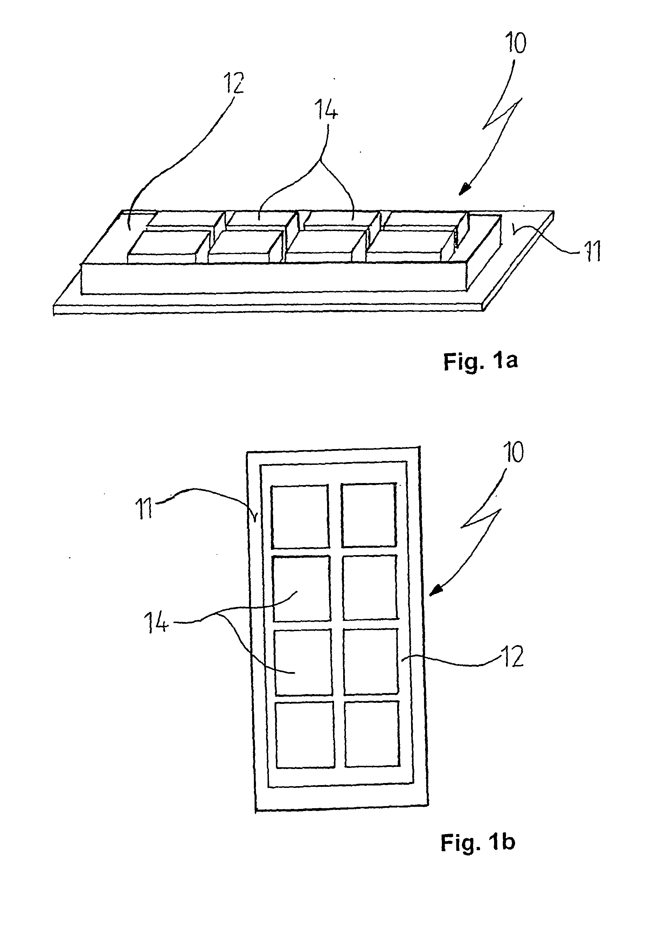

In Fig. 1 ist eine Ausführungsform des erfindungsgemäßen Objektträgers schematisch dargestellt, wobei Fig. 1a eine perspektivische Ansicht und in Fig. 1b eine Draufsicht auf den Objektträger dargestellt ist. In den Figuren sind gleiche Elemente mit den gleichen Bezugszeichen gekennzeichnet. In den Fig. 1a und 1b ist mit 10 allgemein ein Objektträger dargestellt, mit einer Oberfläche 11 und mit einem auf der Oberfläche 11 aufgebrachten Gitteraufsatz 12. Die Oberfläche 11 des Objektträgers 10 wurde vor Aufbringung des Gitteraufsatzes 12 mit Nitrocellulose beschichtet. Der Gitteraufsatz 12 ist an seiner Unterseite mit einer Vaseline-Schicht versehen. Durch Aufbringen des Gitteraufsatzes 12 auf der mit der Nitrocellulose beschichteten Oberfläche 11 entstehen Kammern 14, deren Kontakt untereinander durch die Vaselineschicht unter dem Gitteraufsatz 12 verhindert wird.

In die Mitte der Kammern 14 erfolgte der Auftrag der in Suspension befindlichen Viren (Auftragsvolumen: 1 µl). Die Viren sedimentieren aufgrund ihrer Schwerkraft aus dieser Suspension auf die funktionalisierte Oberfläche, wo sie haften bleiben. Um der Sedimentation und dem Anheften der Viren genügend Zeit einzuräumen, wurden die Objektträger 18 Stunden bei 4°C gehalten. Anschließend wurden die Kammern mit 500 µl PBS gefüllt, um ein Austrocknen zu verhindern. Anschließend wurden die Objektträger bei 37°C gelagert.

Vor Aussaat der Zellen wurden die Kammern mit 500 µl PBS gewaschen, um möglicherweise wieder in Suspension gegangene Viren zu entfernen.

### Beispiel 2

### Stabilität der immobilisierten Viren

Für die praktische Anwendbarkeit war es wichtig zu untersuchen, welche Stabilität die immobilisierten Viren unter gewöhnlichen Umgebungsbedingungen besaßen, da in der Regel davon auszugehen ist, dass eine räumliche und zeitliche Trennung der Immobilisierung der Viren und der Ansiedlung der Zellen erstrebt wird. Deshalb ist die Transport- und Lagerfähigkeit der mit den erfindungsgemäßen Verfahren immobilisierten Viren ein herausragendes Merkmal.

Für den Versuch wurden Viren (AdEasy-CMV-GFP) immobilisiert und nach 7- bzw. 14-tägiger Lagerung bei 37°C jeweils einmal 10⁵ HEK293-Zellen pro Kammer ausgesät. Der Nachweis der erfolgten Infektion erfolgte jeweils einen Tag nach der Aussaat der Zellen durch fluoreszenzmikroskopische Aufnahmen: Infizierte Zellen, die das Gen des GFP aufgenommen hatten, wurden durch Laserlicht zur Fluoreszenz angeregt.

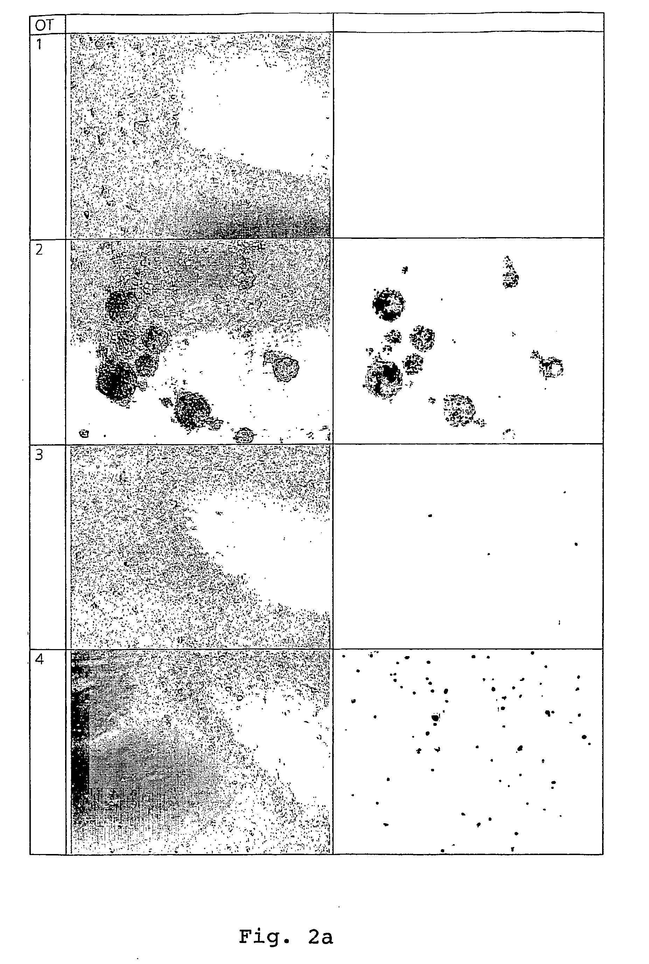

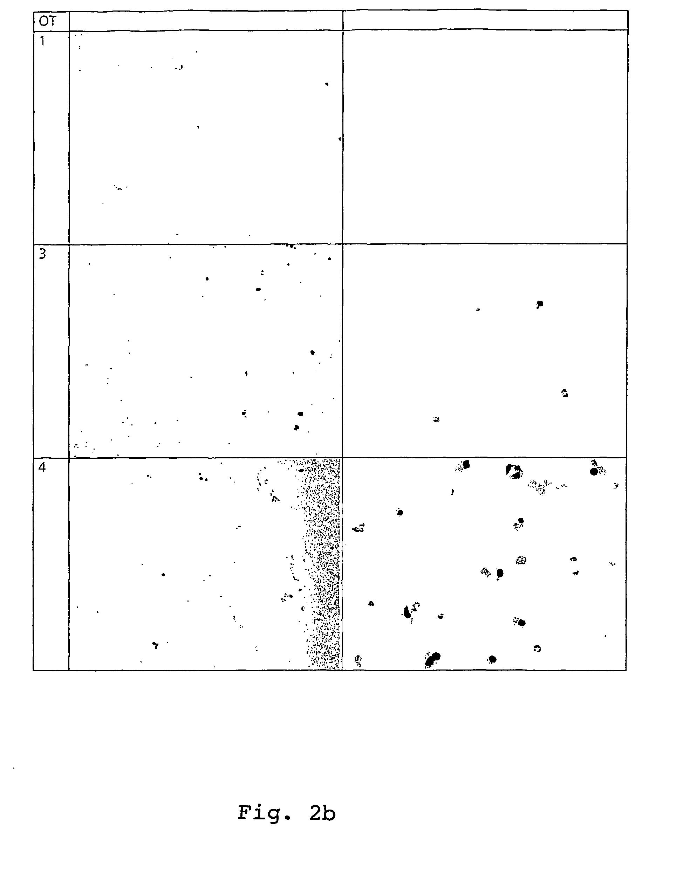

In den Figuren 2a und 2b sind die fluoreszenzmikroskopischen Aufnahmen dieses Versuches dargestellt, wobei in der linken Spalte jeweils vier Objektträger (OT) in Durchlichtaufnahme und in der rechten in Fluoreszenzaufnahme gezeigt sind. Die Fluoreszenzaufnahmen sind zur besseren Darstellung invertiert.

**Tabelle 1**

| **Versuch 1** | | | | | |
|---|---|---|---|---|---|
| Stabilität der immobilisierten Viren | | | | | |
| Objektträger | Virussuspension | Zellauftrag | Fluoreszenz | Zellauftrag | Fluoreszenz |
| | | Tag 7 | Tag 8 | Tag 14 | Tag 15 |
| 1 | --- | HEK293 | - | HEK293 | - |
| 2 | Überstand | HEK293 | + | HEK293 | - |
| 3 | CsCl | HEK293 | +++ | HEK293 | + |
| 4 | CsC1/BSA | HEK293 | +++ | HEK293 | + |

Für den Versuch wurden vier verschiedene Ansätze durchgeführt: Auf einem Objektträger Nr. 1 wurde nur Auftragspuffer ohne Virussuspension aufgetragen (Negativkontrolle). Auf den Objektträgern Nr. 2 bis 4 wurden Viren verschiedener Präparationen immobilisiert: Auf den Objektträger Nr. 2 wurde der Überstand infizierter HEK293-Zellen gegeben, auf den Objektträger Nr. 3 Viren, die über einen Cäsiumchlorid-Gradienten aufgereinigt wurden, und auf den Objektträger Nr. 4 Viren, bei denen auf dem Spot Rinderserumalbumin (BSA, 0,5 µl, 1 mg/ml) vorgelegt wurde.

In der Tabelle 1 sind die Ergebnisse dieses Versuches dargestellt. Adenoviren der verschiedenen Präparationsstufen wurden in den Kammern immobilisiert und die Kammern zu den angegebenen Zeiten (am Tag 7 und am Tag 14 nach der Immobilisierung) mit Zellen beschickt. Im Falle von Cäsiumchlorid/BSA wurden 0,5 µl Rinderserumalbumin (1 mg/ml) vorgelegt und anschließend 1 µl Suspension aufgetragen. Die relativen Fluoreszenzintensitäten sind in Tabelle 1 durch die folgenden Symbole klassifiziert:
- keine Fluoreszenz
+ schwache Fluoreszenz
+++ starke Fluoreszenz.

Bei aus dem Überstand infizierter HEK293-Zellen gewonnenen, nicht weiter aufgereinigten Viren konnte eine Fluoreszenz noch nach 7-tägiger Lagerung festgestellt werden, nicht jedoch, nachdem die Viren 14 Tage gelagert worden waren (siehe Objektträger 2, Zellauftrag nach Tag 7 und nach Tag 14; außerdem in Fig. 2a Objektträger 2 und Fig. 2b Objektträger 2). Die Haltbarkeit bzw. Stabilität der Viren wurde durch die Aufreinigung über die Dichtegradientenzentrifugation deutlich verbessert (siehe Objektträger Nr. 3 in den Figuren 2a und 2b, sowie in der Tabelle 1). Eine darüber hinausgehende Stabilisierung ließ sich durch Vorlage von Rinderserumalbumin erzielen (siehe Objektträger Nr. 4 in den Figuren 2a und 2b sowie in der Tabelle 1).

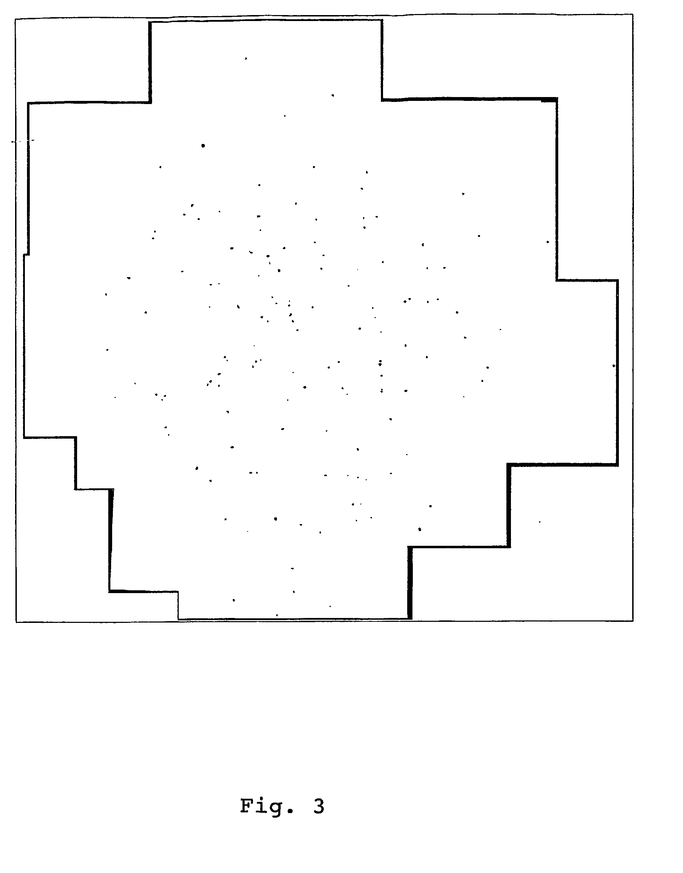

Die Stabilität der Immobilisierung an sich, d.h. die Konstanz der Lokalisation der immobilisierten Viren auf den Spot, wird aus Fig. 3 ersichtlich, die für fiEK 293-Zellen eine Kollage aus verschiedenen Ausschnitten der Fluoreszenz eines Spots einer Kammer am Tag 1 nach der Infektion darstellt: Fluoreszierende Zellen (zur besseren Darstellung invertiert) befinden sich, wie in der Figur zu erkennen ist, hauptsächlich innerhalb der kreisförmigen Spot-Fläche.

Aus diesem Versuch lässt sich demnach schließen, dass die Viren durch ihre Immobilisierung nicht die Fähigkeit verlieren, Zellen zu infizieren und somit fremde genetische Information zu transferieren. Ferner zeigte sich, dass bei der Wahl geeigneter Immobilisierungsbedingungen die Infektiosität über mindestens zwei Wochen bestehen bleibt. Außerdem ist die Immobilisierung der Viren, bzw. die Stabilität ihrer Lokalisierung, innerhalb der ursprünglichen Spot-Fläche gewährleistet.

Die Erfinder konnten demnach zeigen, dass immobilisierte Adenoviren mindestens 14 Tage bei 37°C stabil waren und die Infektion von HEK293-Zellen inklusive Übertragung und Expression des Reportergens GFP ermöglichten.

### Beispiel 3

### Infektion von primären Zellen mit immobilisierten Viren

Wie oben erwähnt, besteht der Vorteil der Verwendung von Viren als Mittel des Gentransfers in den gegenüber den klassischen, physikalischen Methoden deutlich erweitertem Spektrum manipulierbarer Zelltypen. Zu diesen nur viral effizient manipulierbaren Zellen zählen viele Primärzellen, also solche, die entweder nicht kultivierbar sind oder in der Kultur ihre charakteristischen Eigenschaften verlieren. Zur lebensnahen Untersuchung müssen diese Zellen stets frisch aus dem Gewebe isoliert werden, was eine einschneidende Begrenzung der verfügbaren Menge bedingt.

Als Beispiele für Primärzellen wurden in einem nächsten Versuch Mikrogliazellen aus dem Gehirn von 3 Tage alten Ratten eingesetzt. Adenoviren (AdEasy-CMV-GFP) wurden wie oben beschrieben auf beschichtete Objektträger aufgebracht und immobilisiert. Nach 8 Tagen wurden pro Kammer 5 x 10⁴ Zellen (HEK293 als Positivkontrolle, Mikroglia als zu testende primäre Zellen) ausgesät.

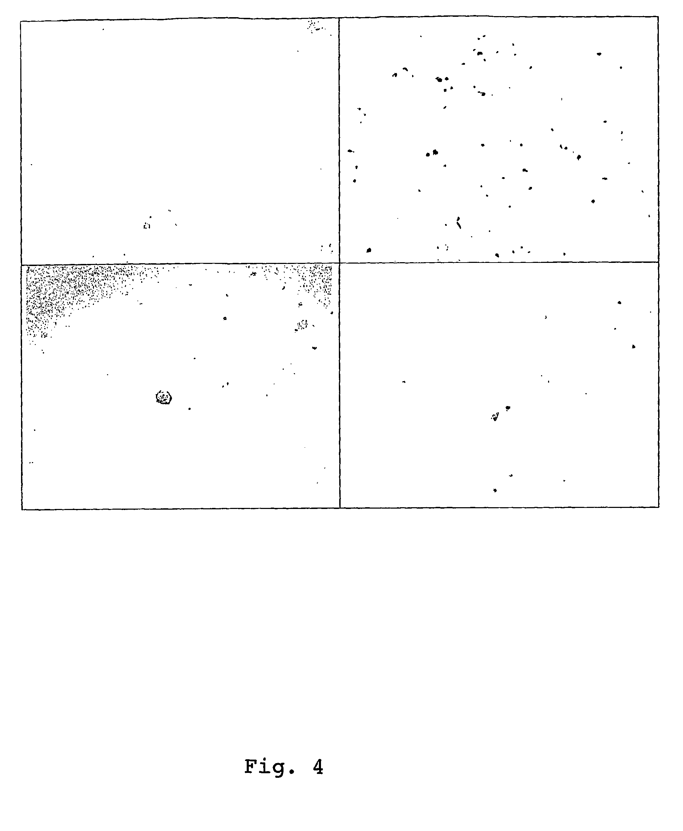

Nach zwei Tagen wurden fluoreszenzmikroskopische Aufnahmen angefertigt (siehe Fig. 4). In der oberen Zeile sind Durchlicht (linke Spalte) und Fluoreszenzaufnahmen (rechte Spalte) für HEK 293-Zellen, in der unteren Zeile für Mikrogliazellen gezeigt. Die Belichtungszeit bei den Fluoreszenzaufnahmen betrug 5 Sekunden. Wie bereits im oben genannten Versuch gezeigt, ließ sich auch hier bei den HEK293-Zellen eine starke Fluoreszenz bei Anregung durch Laserlicht erkennen. Ferner waren auch bei den Mikrogliazellen vereinzelte fluoreszierende Zellen erkennbar.

Dieser Versuch zeigte, dass sich auch Mikrogliazellen, ebenso wie die Kontrollzellen, mit den immobilisierten Viren infizieren ließen. Damit konnte die prinzipielle Anwendbarkeit des Verfahrens auch bei den Primärzellen gezeigt werden, die den klassischen, physikalischen Gentransfermethoden nur schwer zugänglich sind.

### Beispiel 4

### Abschätzung der Infektionseffizienz von primären Zellen mit immobilisierten Viren

Bei vielen Testsystemen ist es insbesondere bei einer kleinen Menge von zu testenden Zellen wichtig, dass die durch die Viren vermittelte genetische Information möglichst viele Zellen erreicht, weil sonst die zu messenden Effekte unterhalb der Nachweisgrenze fallen könnten. Daher ist es von grundsätzlicher Bedeutung, dass die Infektion nicht nur bei einer Minderheit von Zellen gelingt, sondern bei der überwiegenden Mehrheit. Die Effizienz der Infektion ist unter anderem davon abhängig, dass hinreichend viele immobilisierte Viren ihre Infektiosität behalten.

Um die Frage der Infektionseffizienz zu untersuchen, wurde der Versuch der Infektion von Mikrogliazellen wie oben erwähnt wiederholt. Auf zwei zufällig ausgewählten Bildausschnitten wurden bei 200facher Vergrößerung zunächst alle in der durch Lichtaufnahme sichtbaren Zellen gezählt und anschließend die in der Anregung von Laserlicht fluoreszierenden Zellen.

Die Auszählung ergab in einem Fall, dass von 66 Zellen 41 fluoreszierten, in dem anderen Fall fluoreszierten 32 Zellen von gezählten 54 Zellen. Damit lag die Effizienz für die Übertragung und Expression des Reportergens GFP bei Mikrogliazellen in einer Größenordnung von rund 60 %.

Damit konnte gezeigt werden, dass eine Mehrheit der angesiedelten Primärzellen dem viralen Gentransfer zugänglich war. Somit konnte die Einsatzfähigkeit des Verfahrens unter laboralltäglichen Bedingungen demonstriert werden.

Zusammenfassend konnte also gezeigt werden, dass Viren auf einer kleinen Immobilisierungsfläche (Spot) in Rasteranordnung auf funktionalisierten Oberflächen immobilisiert werden konnten und dass ihre Infektiosität für 14 Tage bei 37°C erhalten blieb.

Durch die Infektion konnte das Fremdgen GFP, welches in das virale Genom integriert war, in Zellen übertragen und dort exprimiert werden. Die Infektion gelang nicht nur bei immortalisierten Zellen, wie es die HEK293-Zellen darstellen, sondern auch bei primären Zellen, und zwar mit einer Effizienz von 60 %.

### Beispiel 5: Überprüfung weiterer Oberflächen

Unter den in den Beispielen 1 und 2 geschilderten Bedingungen wurden weitere funktionalisierte Oberfläche auf ihre Eignung getestet, Viren so immobilisieren zu können, dass sie infektiös bleiben.

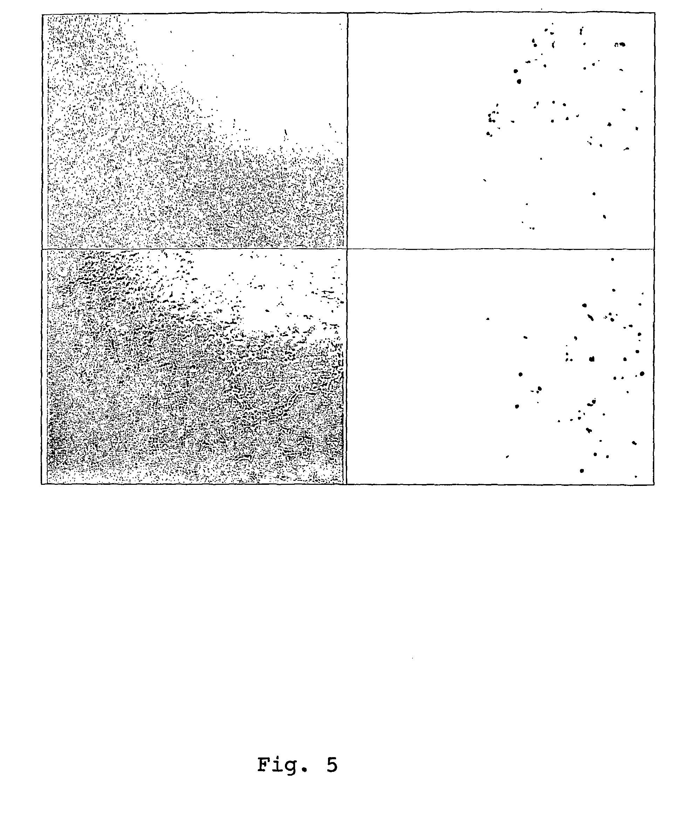

In den Fig. 5-9 zeigt die linke Spalte jeweils wieder Durchlichtaufnahmen, während die rechte Fluoreszenzaufnahmen zeigt, die zur besseren Darstellung invertiert wurden.

Fig. 5 zeigt Aufnahmen für zusätzlich mit Collagen beschichtete Objektträger, die wie in Beispiel 1 beschrieben mit Poly-L-Lysin vorbeschichtete gläserne Objektträger waren, die mit Nitrocellulose beschichtet waren.

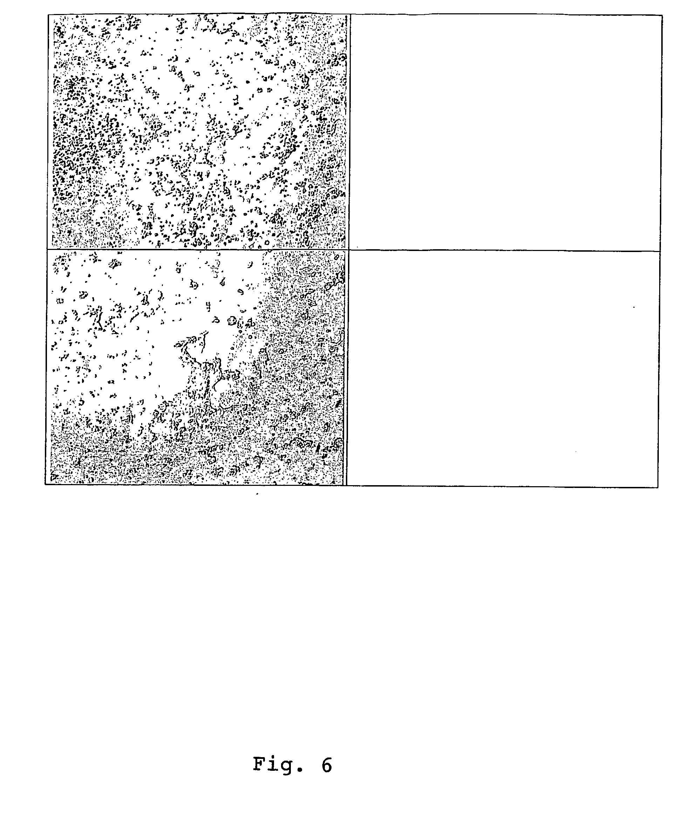

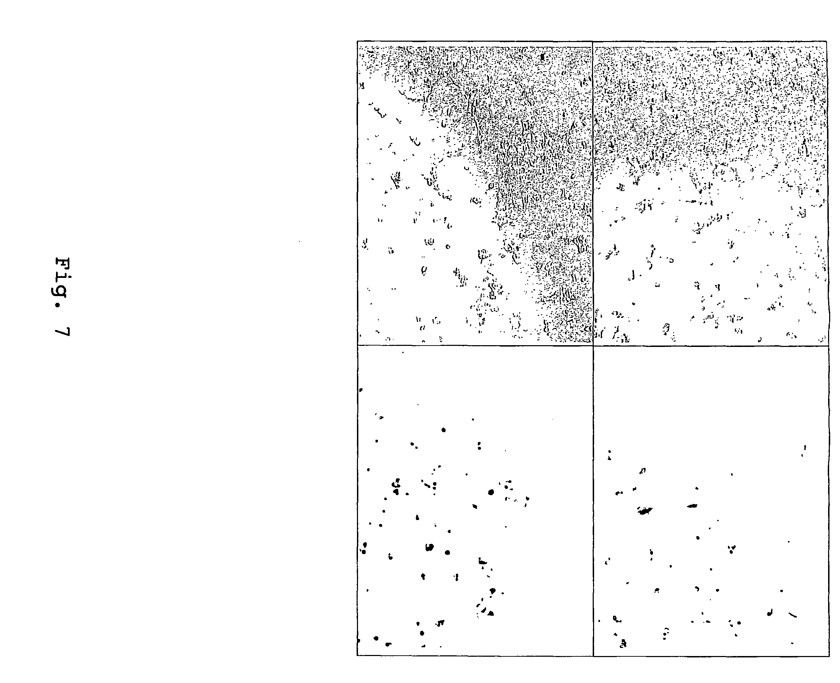

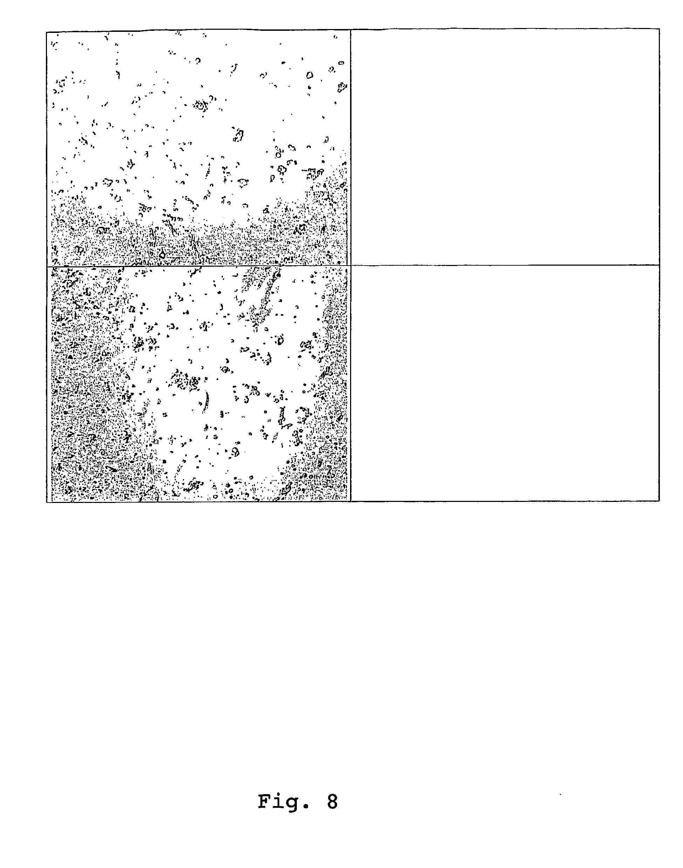

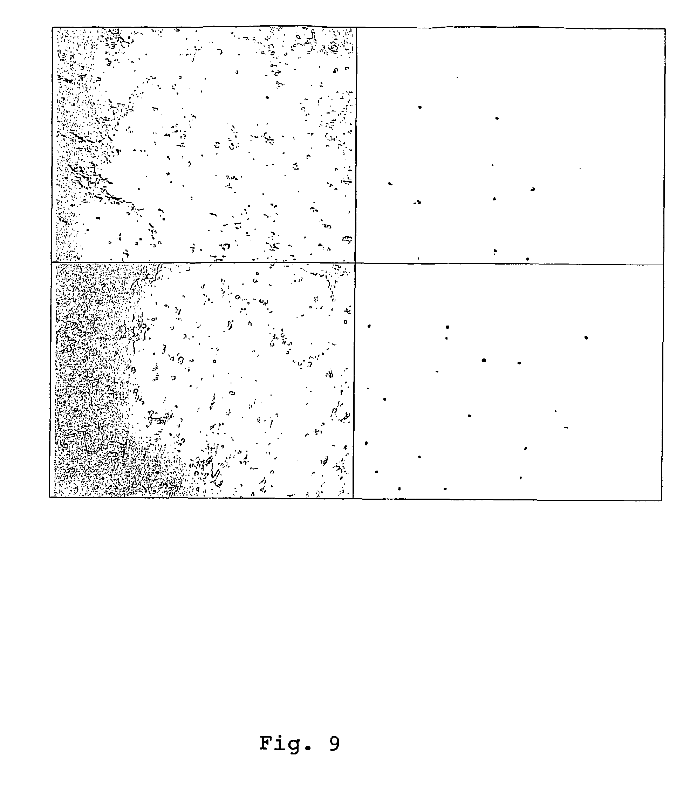

Die Aufnahmen aus Fig. 6 wurden für silanisierte Aldehyd-Objektträger gewonnen, die Aufnahmen aus Fig. 7 unter Verwendung von Superfrost (TM) Plus Adhesion Slides von Electron Microscopy Sciences, die Aufnahmen aus Fig. 8 unter Verwendung von Spot-On (TM) Proteinobjektträgern von Scandinavian Mictrobiodevices, und die Aufnahmen aus Fig. 9 unter Verwendung von GAPS II Coated Slides von Corning.

Während bei den Fig. 5, 7 und 9 in der rechten Spalte jeweils fluoreszierende HEK 293-Zellen zu erkennen waren, ist dies bei den Aufnahmen aus den Fig. 6 und 8 nicht der Fall: Sowohl die silanisierten Aldehyd-Slides aus Fig. 6 als auch die Objektträger aus Fig. 8, bei denen eine kovalente Immobilisierung über Epsilon-Aminogruppen der Proteine erfolgt, zeigen keine Fluoreszenz.

Diese Ergebnisse zeigen, dass die Viren nicht kovalent gekoppelt sein dürfen, um auf dem Objektträger infektiös bleiben zu können.

## Patentansprüche

1. Verfahren zur Herstellung eines Trägers mit einer funktionalisierten Oberfläche und mit auf der funktionalisierten Oberfläche immobilisierten, infektiösen Viren, mit den folgenden Schritten:
(a) Bereitstellen eines Trägermaterials;
(b) Beschichten von zumindest einer Oberfläche des Trägermaterials zur Erzeugung einer für die Immobilisierung von Viren geeigneten funktionalisierten Oberfläche, die mit einem Material beschichtet ist, das die Viren nicht-kovalent bindet;
(c) Aufbringen einer Lösung, welche infektiöse Viren enthält, auf die funktionalisierte Oberfläche;
(d) Anhaftenlassen der Viren an die funktionalisierte Oberfläche für einen Zeitraum von vorzugsweise 10 Minuten bis 36 Stunden;
(e) ggf. Bedecken der Oberfläche mit einem Fluid.

2. Verfahren nach Anspruch 1, **dadurch gekennzeichnet, dass** das Material ausgewählt ist aus Nitrocellulose, Aminosilan und einem Material, das eine dauerhafte positive Ladung auf der Oberfläche bewirkt.

3. Verfahren nach Anspruch 1 oder 2, **dadurch gekennzeichnet, dass** das Trägermaterial ein Material aufweist, das als Oberfläche für Zellkulturen geeignet ist und vorzugsweise aus der Gruppe umfassend Zellkulturkunststoffe, Glas, Silicon, Polystyrol ausgewählt ist.

4. Verfahren nach einem der Ansprüche 1 bis 3, **dadurch gekennzeichnet, dass** das Trägermaterial mit einem Polykation, bspw. mit poly-L-Lysin oder Aminosilan vorbeschichtet ist.

5. Verfahren nach einem der Ansprüche 1 bis 4, **dadurch gekennzeichnet, dass** die Virus-enthaltende Lösung, welche auf die funktionalisierte Oberfläche aufgebracht wird, eine Stoff enthält, der Proteinlösungen stabilisieren kann, wie bspw. Glycerol, Trehalose, und Sacharose.

6. Verfahren nach einem der Ansprüche 1 bis 5, **dadurch gekennzeichnet, dass** die Viren Adenoviren oder Adeno-assoziierte Viren (AAV) sind.

7. Verfahren nach Anspruch 6, **dadurch gekennzeichnet, dass** die Adenoviren den Serotyp 5 aufweisen.

8. Verfahren nach einem der Ansprüche 1 bis 7, **dadurch gekennzeichnet, dass** die Viren gentechnisch verändert sind.

9. Verfahren nach Anspruch 8, **dadurch gekennzeichnet, dass** die Viren eine Sequenz aufweisen, die für das Green-fluorescent-Protein codiert.

10. Verfahren nach einem der Ansprüche 1 bis 9, **dadurch gekennzeichnet, dass** als Virus-enthaltende Lösung ein Überstand Virus-infizierter Zellen eingesetzt wird.

11. Verfahren nach Anspruch 10, **dadurch gekennzeichnet, dass** der Überstand vor Aufbringen auf die funktionalisierte Oberfläche gereinigt und /oder aufkonzentriert wird.

12. Verfahren nach einem der Ansprüche 1 bis 11, **dadurch gekennzeichnet, dass** vor Schritt (c) Rinderserumalbumin auf die funktionalisierte Oberfläche aufgebracht wird.

13. Verfahren nach einem der Ansprüche 1 bis 12, **dadurch gekennzeichnet, dass** der Zeitraum, in dem man die Viren auf der funktionalisierten Oberfläche anhaften lässt, 15 bis 20 Stunden beträgt.

14. Verfahren nach einem der Ansprüche 1 bis 13, **dadurch gekennzeichnet, dass** die Temperatur, bei welcher man die Viren an die funktionalisierte Oberfläche anhaften lässt, -20°C bis +37°C beträgt.

15. Verfahren nach Anspruch 14, **dadurch gekennzeichnet, dass** die Temperatur 0°C bis 10°C beträgt.

16. Träger mit einer für die Immobilisierung von Viren geeigneten funktionalisierten Oberfläche und mit auf der funktionalisierten Oberfläche durch nicht-kovalente Bindung immobilisierten, infektiösen Viren.

17. Verwendung eines Trägers mit einer für die Immobilisierung von Viren geeigneten funktionalisierten Oberfläche und mit auf der funktionalisierten Oberfläche durch nicht-kovalente Bindung immobilisierten infektiösen Viren zum Transfer genetischen Materials in Zellen, mit den folgenden Schritten:
(a) Bereitstellen eines Trägers mit einer für die Immobilisierung von Viren geeigneten funktionalisierten Oberfläche und mit auf der funktionalisierten Oberfläche durch nicht-kovalente Bindung immobilisierten infektiösen Viren;
(b) In-Kontakt-Bringen des Trägers mit einer Suspension, welche Zellen enthält.

18. Verwendung nach Anspruch 17, **dadurch gekennzeichnet, dass** die Zellen pro- bzw. eukaryontische Zellen sind.

19. Verwendung nach Anspruch 18, **dadurch gekennzeichnet, dass** die Zellen primäre eukaryontische Zellen sind.

20. Verwendung nach Anspruch 18, **dadurch gekennzeichnet, dass** die Zellen immortalisierte Zellen sind.

21. Verwendung nach Anspruch 18, **dadurch gekennzeichnet, dass** die Viren Bakteriophagen sind.

22. Verwendung nach einem der Ansprüche 17 bis 21, **dadurch gekennzeichnet, dass** ein Träger eingesetzt wird, der nach einem der Ansprüche 1 bis 15 hergestellt ist.

23. Verfahren zur Einschleusung von DNA oder RNA in eine pro- oder eukaryontische Zelle, mit den Schritten:
(a) Aufbringen einer Virus-enthaltenden Lösung auf einen Träger mit einer für die Immobilisierung von Viren geeigneten funktionalisierten Oberfläche, die mit einem Material beschichtet ist, das die Viren nicht-kovalent bindet,
(b) Anhaftenlassen der Lösung auf der Oberfläche,
(c) Aufbringen einer Suspension, welche Zellen enthält, auf die Oberfläche, um die Zellen mit der in den Viren enthaltenen DNA oder RNA zu transduzieren, bei dem ein Träger verwendet wird, der nach einem der Ansprüche 1 bis 15 hergestellt ist.

24. Verfahren zum Lagern von infektiösen Viren, **dadurch gekennzeichnet, dass** sie auf einem Träger mit einer funktionalisierten Oberfläche immobilisiert werden, die mit einem Material beschichtet ist, das die Viren nicht-kovalent bindet, wobei ein Träger verwendet wird, der nach einem der Ansprüche 1 bis 15 hergestellt ist.

## Claims

1. Method for producing a support having a functionalized surface and having infectious viruses, which are immobilized on the functionalized surface, comprising the following steps:
(a) providing a support material;
(b) coating at least one surface of the support material for the purpose of producing a functionalized surface which is suitable for immobilizing viruses, which functionalized surface is coated with a material which binds the viruses noncovalently.
(c) applying a solution, which contains infectious viruses, to the functionalized surface;
(d) leaving the viruses to adhere to the functionalized surface over a period of from preferably 10 minutes to 36 hours;
(e) where appropriate, covering the surface with a fluid.

2. Method according to Claim 1, **characterized in that** the material is selected from nitrocellulose, aminosilane and a material which causes a lasting positive charge on the surface.

3. Method according to Claim 1 or 2, **characterized in that** the support material comprises a material which is suitable for use as a surface for cell cultures and is preferably selected from the group comprising cell culture plastics, glass, silicone and polystyrene.

4. Method according to anyone of Claims 1 to 3, **characterized in that** the support material is precoated with a polycation, for example with poly-L-lysine or aminosilane.

5. Method according to anyone of Claims 1 to 4, **characterized in that** the virus-containing solution, which is applied to the functionalized surface, contains a substance which can stabilize protein solutions, such as glycerol, trehalose and sucrose.

6. Method according to anyone of Claims 1 to 5, **characterized in that** the viruses are adenoviruses or adeno-associated viruses (AAVs).

7. Method according to Claim 6, **characterized in that** the adenoviruses are of serotype 5.

8. Method according to anyone of Claims 1 to 7, **characterized in that** the viruses have been genetically modified.

9. Method according to Claim 8, **characterized in that** the viruses comprise a sequence which encodes for the Green Fluorescent Protein.

10. Method according to anyone of Claims 1 to 9, **characterized in that** a supernatant from virus-infected cells is employed as the virus-containing solution.

11. Method according to Claim 10, **characterized in that** the supernatant is purified and/or concentrated before being applied to the functionalized surface.

12. Method according to anyone of Claims 1 to 11, **characterized in that** bovine serum albumin is applied to the functionalized surface prior to step (c).

13. Method according to anyone of Claims 1 to 12, **characterized in that** the period in which the viruses are left to adhere to the functionalized surface is from 15 to 20 hours.

14. Method according to anyone of Claims 1 to 13, **characterized in that** the temperature at which the viruses are left to adhere to the functionalized surface is from -20°C to +37°C.

15. Method according to Claim 14, **characterized in that** the temperature is from 0°C to 10°C.

16. Support having a functionalized surface which is suitable for immobilizing viruses and having infectious viruses which are immobilized on the functionalized surface by noncovalent binding.

17. Use of a support having a functionalized surface which is suitable for immobilizing viruses and having infectious viruses which are immobilized on the functionalized surface by noncovalent binding, for the transfer of genetic material into cells, comprising the following steps:
(a) providing a support having a functionalized surface which is suitable for immobilizing viruses and having infectious viruses which are immobilized on the functionalized surface by noncovalent binding;
(b) bringing the support into contact with a suspension which contains cells.

18. Use according to claim 17, **characterized in that** the cells are prokaryotic and eukaryotic cells, respectively.

19. Use according to claim 18, **characterized in that** the cells are primary eukaryotic cells.

20. Use according to claim 18, **characterized in that** the cells are immortalized cells.

21. Use according to Claim 18, **characterized in that** the viruses are bacteriophages.

22. Use according to anyone of Claims 17 to 21, **characterized in that** use is made of a support which has been produced according to the method of anyone of Claims 1 to 15.

23. Method for inserting DNA or RNA into a prokaryotic or eukaryotic cell, comprising the steps of:
(a) applying a virus-containing solution to a support having a functionalized surface which is suitable for immobilizing viruses, which surface is coated with a material which binds the viruses noncovalently.
(b) allowing the solution to adhere to the surface,
(c) applying a suspension, which contains cells, to the surface in order to transduce the cells with the DNA or RNA which is contained in the viruses, wherein use is made of a support which has been produced according to the method of anyone of Claims 1 to 15.

24. Method for storing infectious viruses, **characterized in that** they are immobilized on a support having a functionalized surface coated with a material which binds the viruses noncovalently, whereby use is made of a support which has been produced according to the method of anyone of Claims 1 to 15.

## Revendications

1. Procédé pour la fabrication d'un support présentant une surface fonctionnalisée et des virus infectieux immobilisés sur la surface fonctionnalisée, le procédé comprenant les étapes suivantes :
(a) la préparation d'un matériau de support ;
(b) le dépôt d'un revêtement sur au moins une surface du matériau de support, dans le but de produire une surface fonctionnalisée appropriée pour l'immobilisation des virus, la surface en question étant revêtue d'un matériau auquel les virus se lient de manière non covalente ;
(c) l'application d'une solution contenant des virus infectieux à la surface fonctionnalisée ;
(d) l'adhérence des virus à la surface fonctionnalisée sur une période de temps dans la plage, de préférence, de 10 min à 36 heures ;
(e) éventuellement, le recouvrement de la surface avec un fluide.

2. Procédé selon la revendication 1, **caractérisé en ce que** le matériau est choisi parmi la nitrocellulose, l'aminosilane et un matériau qui génère une charge positive permanente à la surface.

3. Procédé selon la revendication 1 ou 2, **caractérisé en ce que** le matériau de support présente un matériau qui soit approprié comme surface de culture cellulaire et que l'on choisit, de préférence, dans le groupe comprenant les matériaux synthétiques destinés à la culture cellulaire, le verre, la silicone et le polystyrène.

4. Procédé selon l'une quelconque des revendications 1 à 3, **caractérisé en ce que** le matériau de support est préalablement revêtu d'un polycation, par exemple, de poly-L-lysine ou d'aminosilane.

5. Procédé selon l'une quelconque des revendications 1 à 4, **caractérisé en ce que** la solution contenant le virus, qui est appliquée sur la surface fonctionnalisée, contient une substance qui peut stabiliser les solutions de protéines, par exemple, du glycérol, du tréhalose et du saccharose.

6. Procédé selon l'une quelconque des revendications 1 à 5, **caractérisé en ce que** les virus sont des adénovirus ou des virus adéno-associés (AAV).

7. Procédé selon la revendication 6, **caractérisé en ce que** les adénovirus présentent le sérotype 5.

8. Procédé selon l'une quelconque des revendications 1 à 7, **caractérisé en ce que** les virus sont génétiquement modifiés.

9. Procédé selon la revendication 8, **caractérisé en ce que** les virus présentent une séquence qui code pour la protéine à fluorescence verte.

10. Procédé selon l'une quelconque des revendications 1 à 9, **caractérisé en ce que** l'on utilise comme solution contenant le virus, un surnageant de cellules infectées par le virus.

11. Procédé selon la revendication 10, **caractérisé en ce que** le surnageant est purifié et/ou concentré avant d'être appliqué sur la surface fonctionnalisée.

12. Procédé selon l'une quelconque des revendications 1 à 11, **caractérisé en ce que** l'on applique de l'albumine de sérum bovin sur la surface fonctionnalisée avant de procéder à l'étape (c).

13. Procédé selon l'une quelconque des revendications 1 à 12, **caractérisé en ce que** la période de temps au cours de laquelle on laisse les virus adhérer à la surface fonctionnalisée, s'étend de 15 à 20 heures.

14. Procédé selon l'une quelconque des revendications 1 à 13, **caractérisé en ce que** la température à laquelle on laisse les virus adhérer à la surface fonctionnalisée, se situe dans la plage de -20 °C à +37 °C.

15. Procédé selon la revendication 14, **caractérisé en ce que** la température se situe dans la plage de 0 °C à 10 °C.

16. Support présentant une surface fonctionnalisée convenant à l'immobilisation de virus, et présentant des virus infectieux immobilisés à la surface fonctionnalisée par le biais de liaisons non covalentes.

17. Utilisation d'un support présentant une surface fonctionnalisée convenant à l'immobilisation de virus, et présentant des virus infectieux immobilisés sur la surface fonctionnalisée, via des liaisons non covalentes, dans le but de transférer un matériau génétique dans des cellules, comprenant les étapes suivantes .
(a) on prépare un support présentant une surface fonctionnalisée convenant à l'immobilisation de virus, et présentant des virus infectieux immobilisés à la surface fonctionnalisée par le biais de liaisons non covalentes ;
(b) on met le support en contact avec une suspension qui contient des cellules.

18. Utilisation selon la revendication 17, **caractérisée en ce que** les cellules sont des cellules procaryotes ou eucaryotes.

19. Utilisation selon la revendication 18, **caractérisée en ce que** les cellules sont des cellules eucaryotes primitives.

20. Utilisation selon la revendication 18, **caractérisée en ce que** les cellules sont des cellules immortalisées.

21. Utilisation selon la revendication 18, **caractérisée en ce que** les virus sont des bactériophages.

22. Utilisation selon l'une quelconque des revendications 17 à 21, **caractérisée en ce que** l'on utilise un support qui a été fabriqué selon l'une quelconque des revendications 1 à 15.

23. Procédé pour introduire de l'ADN ou de l'ARN dans une cellule procaryote ou eucaryote, comprenant les étapes suivantes :
(a) on applique une solution contenant le virus sur un support présentant une surface fonctionnalisée convenant à l'immobilisation des virus, surface que l'on a revêtue d'un matériau auquel les virus se lient de manière non covalente,
(b) on laisse la solution adhérer à la surface,
(c) on applique une suspension, qui contient des cellules, à la surface dans le but de réaliser la transduction des cellules avec l'ADN ou l'ARN contenu dans les virus, en utilisant un support qui a été fabriqué selon l'une quelconque des revendications 1 à 15.

24. Procédé pour stocker des virus, de préférence, infectieux, **caractérisé en ce que** ceux-ci sont immobilisés sur un support présentant une surface fonctionnalisée qui a été revêtue d'un matériau auquel les virus se lient de manière non covalente, en utilisant un support qui a été fabriqué selon l'une quelconque des revendications 1 à 15.
